# EUROPEAN PATENT APPLICATION

(11) **EP 4 556 002 A1**
(43) Date of publication of application: **21.05.2025**
(21) Application number: 22950984.9
(22) Date of filing: 14.07.2022
(51) Int. Cl.: A61K 9/68, A23G 4/06

(54) **VIRUCIDAL COMPOSITIONS AND USES THEREOF**

(71) Applicant: Delicatessen Ideas Agitadas S.L., 08012 Barcelona (ES)
(72) Inventor: GONZÁLEZ-CUEVAS LABELLA, Jose Antonio, 08012 Barcelona (ES); ESPINÓS DE PASCUAL, Francisco, 08012 Barcelona (ES); ISAMAT RIVIÈRE, Marcos, 08012 Barcelona (ES)
(74) Representative: Balder IP Law, S.L.
(86) International application number: PCT/ES2022/070458
(87) International publication number: WO 2024/013406

(57) **Abstract**

The invention discloses a composition comprising acetic acid, phosphoric acid and citric acid which shows high virucidal activity. In some embodiments, it comprises 1 to 30% by weight of acetic acid, 0.2 to 10% by weight of citric acid and 0.01 to 1.0% by weight of phosphoric acid. The composition can be used for manufacturing various edible products, such as candies and gums, and non-edible products such as toothpastes, shampoos, etc., or products for disinfecting cloths, tools, air, etc. More particularly, the invention discloses a composition comprising acetic, citric and phosphoric acids in chewing gum for use thereof as a medicinal product and for use thereof in the treatment of viral and/or bacterial infections, in which the virus is an enveloped virus, such as coronaviruses.

## Description

### TECHNICAL FIELD OF THE INVENTION

The present invention relates to virucidal chewing gum which incorporates a combination of acids suitable for consumption and which also has optimized organoleptic properties.

### BACKGROUND OF THE INVENTION

Viral epidemics typically develop from the arrival of new variants of known infectious viruses. Virus transmission can occur at unprecedented rates through human-to-human contact, or contact with contaminated surfaces, and it is essential to find effective therapies, as well as disinfecting or sanitizing agents.

It is known that depending on the type of contaminated surface and environmental conditions, viruses can persist on the surface for at least 5 minutes and up to 28 days. As illustrated by Kampf et al. (J. Hosp. Infect. 2020, 104(3), 246-251) human coronaviruses such as SARS (Severe Acute Respiratory Syndrome) coronavirus, Middle East Respiratory Syndrome (MERS) coronavirus or human endemic coronaviruses (HCoV) persist on inert surfaces such as metal, glass or plastic for up to 9 days. In these cases, a 5-second contact between hand and surface may be enough to transfer a significant portion of virus that can be spread by touching the oral mucosa, nasal mucosa or conjunctiva of the eye.

As regards treatments against viral infections, most of them include antiviral agents intended for the parenteral route of administration, particularly in the form of capsules or tablets. In the most severe cases, the parenteral route of administration provides an effective alternative for easy administration of higher doses of the antiviral medicinal product. Furthermore, combined administration of different antiviral agents in the form of tablets is another typically preferred option, particularly for patients who do not respond to typical antiviral monotherapies.

However, these therapeutic options usually cause severe side effects, particularly in the case of medicinal products administered at high doses. For example, oral administration of antiviral agents in the form of tablets can lead to short- or long-term side effects, when administered at high doses, which can affect the digestive system. This can lead to reduced patient compliance, particularly in those cases where prolonged treatments are required.

Furthermore, the combined administration of antiviral agents orally, particularly in the form of tablet, or even parenterally, can result in drug interactions, which could cause greater long-term side effects in the patient.

Unlike antiviral drugs, which typically destroy the virus's ability to replicate, a different type of compounds known as virucidal agents directly inactivate the virus such that it cannot infect host cells through modification of the surface structures of the virus or destruction of the virus. However, virucidal agents such as bleach, hydrochloric anhydride or even soapy water are not suitable for use on the human body due to potential health risks. Their use is therefore restricted to surface disinfection. However, given the potential efficacy of virucidal agents against viral infections in humans, it is desirable to find new agents of this type with a similar mode of action but free of the drawbacks associated with conventional virucidal agents.

In this scenario, there is a clear need for new effective virucides that significantly reduce the risk of virus spreading through contact with infected surfaces or aerosols which may reach the oral/nasal environment and that can be used in therapeutic applications, particularly for the treatment of infected patients. Furthermore, in turn, it is desirable to obtain a new virucidal formulation in which the side effects typically associated with classical routes of administration of antiviral drugs are drastically reduced.

Moreover, gum is known, particularly from document US20070237805A1, which relates to chewing gums and confectionery compositions for improving the dental health of mammals, particularly humans. In particular, the compositions described in said document may include a gum base or carrier, sweetening agents, casein phosphopeptide-calcium phosphate (CPP-ACP) and food-grade acids. The addition of flavors, many of which are acidic in nature, is an everyday occurrence in the manufacture of gum, but this poses a risk for the development or aggravation of dental caries. For this reason, according to this document, compounds intended for remineralizing tooth surfaces of mammals are added.

### DETAILED DESCRIPTION OF THE INVENTION

To overcome the drawbacks of state of the art, the present invention proposes chewing gum containing:
1 to 30% w/v acetic acid,
0.2 to 10% w/v citric acid,
0.01 to 1.0% w/v phosphoric acid, and
wherein the % w/v is with respect to the weight of the total volume of the composition.

The virucidal chewing gum object of the invention solves the drawbacks of the state of the art. On one hand, it has demonstrated a significant virucidal capacity by means of the combination of GRAS acids.

In some embodiments, acetic acid is included in an amount of 2 to 3% w/v and is preferably 2.5%; citric acid is included in an amount of 1.5 to 2% w/v and is preferably 1.75%; and phosphoric acid is included in an amount of 0.05 to 0.07% w/v, and is preferably 0.06%.

In particular, it has been verified that this combination of acids with chewing gum at the specified concentrations lead to release rates that are effectively virucidal when chewing the gum. However, in combination with this capacity, the concentrations have been shown to be acceptable for most users from an organoleptic viewpoint, an essential condition for the user to keep the gum in his/her mouth continuously, leading to the effect that is sought.

This particular selection corresponds to the composition containing the least added acids, i.e., whose sum of acetic acid, citric acid and phosphoric acid is lower, which has proven to be acceptable by everyone who has tried the composition, all this while maintaining the virucidal capacity at about 99%.

Furthermore, the claimed set has proven to be very stable under storage conditions.

The chewing gum may comprise:
- elastomeric polymers, preferably specially purified elastomeric polymers;
- resins;
- refined waxes;
- plant fatty acid glycerol esters;
- fully hydrogenated vegetable oils;
- talc;
- antioxidant, preferably TCPHN in a maximum amount of 0.1%.

With these components being known, the person skilled in the art will know how to choose the concentration ranges of each of them.

The composition may further incorporate optionally one or more chewing gum components such as sweeteners, sugars, flavorings, taste-masking agents and vitamins.

The base gum is retained in the mouth during the chewing period and generally contains elastomers, resins, fats, oils, waxes, softeners and inorganic fillers. Examples of suitable elastomers may include, but are not limited to, for example, polyisobutylene, isobutylene-isoprene co-polymer, styrene-butadiene rubber, and also natural latex types. Suitable resins may include, but are not limited to, the following examples: terpene and polyvinyl acetate resins. Illustrative fats and oils may include, but are not limited to, tallow, hydrogenated or partially hydrogenated vegetable oils, or coconut butter. Typically used waxes include paraffin, natural and microcrystalline waxes such as beeswax or carnauba. A typical inorganic filler may be talc. The base gum may optionally contain additionally at least one softener such as glycerol monostearate or glycerol triacetate. The base gum may also contain additional ingredients such as antioxidants, colorants and/or emulsifiers. All commercial base gums, accepted as safe for human consumption, can be contemplated for the purpose of the composition of the present invention.

Advantageously, the chewing gum incorporates one or more components such as sweeteners, sugars, flavorings, taste-masking agents and vitamins.

Sweeteners may be added to the chewing gum to increase sweetness perception. Examples of suitable sweeteners include, but are not limited to, sorbitol, xylitol, mannitol or maltitol.

As mentioned above, the chewing gum composition may further contain at least one sugar, such as glucose, sucrose, fructose, invert sugar, maltose, lactose or any mixture thereof.

Flavorings which may be added to the chewing gum composition can result in various flavors. Particularly preferred flavorings are liquids or powder and also encapsulated flavors. Suitable flavorings include, but are not limited to, mint, vanilla, strawberry, orange or citrus flavors. Furthermore, they can provide the advantage of masking the taste of the composition, even the gelatinous taste.

Suitable vitamins in the chewing gum composition, and according to some embodiments, include vitamin C, vitamin B2, vitamin B6, vitamin B12, vitamin E, vitamin D, vitamin A, biotin, niacin, thiamin, panthothenic acid, folic acid or any mixture thereof.

Taste-masking agents can conceal a specific aspect of the taste of the chewing gum composition, such as a sour taste. In some embodiments, the taste-masking agents comprise at least one flavoring and/or sweetener as indicated above. Some suitable high-intensity taste-masking agents include polyol sweeteners which may be selected from a list consisting of dextrose, sucrose, maltose and lactose. In some embodiments, suitable high-intensity taste-masking agents may be selected from the group consisting of sucralose, neotame, aspartame, acesulfame salts, alitame, saccharin and salts thereof, cyclamic acid and salts thereof, glycitin, dihydrochalcones such as NHDC), thaumatin, monellin, stevioside, aspartame-acesulfame salt and any mixture thereof. In a specific embodiment, the taste-masking agent has been selected from the group consisting of dextrose, sucrose, fructose, lactose, sucralose, neotame, aspartame, acesulfame salts, alitame, saccharin and salts thereof, cyclamic acid and salts thereof, glycyrrhizin, dihydrochalcones (such as NHDC), thaumatin, monellin, stevioside, aspartame-acesulfame salt and any combination thereof.

The chewing gum may be manufactured according to all conventional methods. As an illustration, it can be produced by directly mixing the base gum with acetic acid, citric acid and phosphoric acid and any other component defined above.

In an alternative embodiment, the chewing gum may be manufactured by sequential or simultaneous addition of the various ingredients in a commercial mixer known in the art. After the ingredients have been mixed, the gum mass is removed from the mixer and molded into the desired shape, for example with rollers to produce sheets, and cutting same into slices, extruding same into pieces or molding same into shapes or tablets. The ingredients can be mixed after melting the base gum and adding it to the running mixer. A flavoring agent can be melted inside the same mixer. Additional ingredients can also be added at this time. Additional portions of sweeteners, for example, can be added to the mixer. A high-intensity flavoring agent is typically added to the final portion. A high-intensity sweetener can preferably be added in subsequent production steps, after the flavor has been added.

Throughout the description and claims, the word "comprises" or "consists" or variations of both, do not exclude other technical terms, ingredients or steps. Additional advantages and features of the invention will be apparent to those skilled in the art from their examination of the description or may be learned by practicing the invention without undue burden.

### DESCRIPTION OF PREFERRED EMBODIMENTS OF THE INVENTION

The following experimental examples and results are provided by way of non-limiting illustration and cannot be considered as limiting the invention.

### EXAMPLE 1

### Study of the virucidal properties of the chewing gum according to the invention

Virucidal activity in the context of the present invention is to be understood as the inactivation property of the composition. The composition of the first aspect of the invention contains: acetic acid, citric acid and phosphoric acid.

Virucidal activity has been measured according to EN 14476:2013+A2: 2019 standard under clean conditions and on modified vaccinia virus Ankara (MVVA) which validates the results of virucidal activity against all enveloped viruses, covering enveloped viruses such as HBV, HCV, HIV, as well as viruses from other families such as Orthomyxoviridae (which include all human influenza viruses), Coronaviridae (which include MERS-CoV, SARS-CoV-1 and SARS-CoV-2) and Filoviridae including Ebola virus (Eggers M et al., Povidone-iodine hand wash and hand rub products demonstrated excellent in vitro virucidal efficacy against Ebola virus and modified vaccinia virus Ankara, the new European test virus for enveloped viruses. BMC Infect Dis. 2015; 15:375).

This test demonstrates that 99.99% of all viral particles are destroyed and not capable of infecting BHK-21 cells in culture at 37°C after 1 minute exposure to the following compositions (undiluted, 50% diluted, 20% diluted and 10% diluted):
VS1 (virucidal solution 1): consisting of:
   - Acetic acid at a concentration of 525 mg/ml (or 52.5% or 52.5 g/100 ml);
   - Citric acid at a concentration of 20 mg/ml (or 2% or 2 g/100 ml);
   - Phosphoric acid at a concentration of 6 mg/ml; (or 0.6% or 0.6 g/100 ml);
   - Water.
VS2 (virucidal solution 2): 50% diluted consisting of:
   - Acetic acid at a concentration of 262 mg/ml (or 26.25% or 26.25 g/100 ml);
   - Citric acid at a concentration of 10 mg/ml (or 1% or 1 g/100 ml);
   - Phosphoric acid at a concentration of 3 mg/ml (or 0.3% or 0.3 g/100 ml).
VS3 (virucidal solution 3): 20% diluted consisting of:
   - Acetic acid at a concentration of 105 mg/ml (or 10% or 10 g/100 ml);
   - Citric acid at a concentration of 4 mg/ml (or 4% or 4 g/100 ml);
   - Phosphoric acid at a concentration of 1.2 mg/ml (or 0.12% or 0.12 g/100 ml);
   - Water.
VS4 (virucidal solution 4): 10% diluted consisting of:
   - Acetic acid at a concentration of 52.5 mg/ml (or 5.25% or 5.25 g/100 ml);
   - Citric acid at a concentration of 2 mg/ml (or 2% or 2 g/100 ml);
   - Phosphoric acid at a concentration of 0.6 mg/ml (or 0.06% or 0.06 g/100 ml);
   - Water.
VS5 (virucidal solution 5): 0.05% diluted consisting of:
   - Acetic acid at a concentration of 0.26 mg/ml (or 0.02% or 0.02 g/100 ml);
   - Citric acid at a concentration of 0.01 mg/ml (or 0,001% or 0,001 g/100 ml);
   - Phosphoric acid at a concentration of 0,003 mg/ml (or 0.0003% or 0.0003 g/100 ml);
   - Water.

The 0.05% dilution showed some virucidal activity after 1-minute exposure to BHK-21 cells and was therefore considered non-virucidal at this dilution factor.

Another test for the virucidal activity of the composition consists of:
VS6 (virucidal solution 6) consisting of:
- Acetic acid at a concentration of 250 mg/ml (or 25% or 25 g/100 ml);
- Citric acid at a concentration of 10 mg/ml (or 1% or 1 g/100 ml);
- Phosphoric acid at a concentration of 30 mg/ml (or 3% or 3 g/100 ml);
- Water.

This test was performed directly on SARS-CoV-2 GT4CNS virus exposed to the composition for 5 minutes and incubated with VeroE6 cells, giving a 97 to 99% (99.9%?) reduction in the infectivity of VeroE6 cells by SARS-CoV-2 virus.

Another test for the virucidal activity of the composition consists of:
VS7 (virucidal solution 7) consisting of:
- Acetic acid at a concentration of 52.5 mg/ml (or 5.25% or 5.25 g/100 ml);
- Citric acid at a concentration of 2 mg/ml (or 0.2% or 0.2 g/100 ml);
- Phosphoric acid at a concentration of 0.6 mg/ml (or 0.6% or 0.6 g/100 ml);
- Water.

This test was performed directly on SARS-CoV-2 GT4CNS virus exposed to the composition for 5 minutes and incubated with VeroE6 cells, giving a 97 to 99% reduction in the infectivity of VeroE6 cells by SARS-CoV-2 virus.

Another test for the virucidal activity of the composition consists of:
VS8 (virucidal solution 8) consisting of:
- Acetic acid at a concentration of 25 mg/ml (or 2.5% or 2.5 g/100 ml);
- Citric acid at a concentration of 17.5 mg/ml (or 1.75% or 1.75 g/100 ml);
- Phosphoric acid at a concentration of 0.6 mg/ml (or 0.06% or 0.06 g/100 ml);
- Water.

This test was performed directly on SARS-CoV-2 GT4CNS virus exposed to the composition for 5 minutes and incubated with VeroE6 cells, giving a 97 to 99% reduction in the infectivity of VeroE6 cells by SARS-CoV-2 virus.

Another test for the virucidal activity of the composition consists of:
VS9 (virucidal solution 9) consisting of:
- Sodium acetate at a concentration of 71.7 mg/ml (or 7.17% or 7.17 g/100 ml);
- Citric acid at a concentration of 2 mg/ml (or 0.2% or 0.2 g/100 ml);
- Phosphoric acid at a concentration of 0.6 mg/ml (or 0.06% or 0.06 g/100 ml);
- Water.

This test was performed directly on SARS-CoV-2 GT4CNS virus exposed to the composition for 5 minutes and incubated with VeroE6 cells, giving a 40% reduction in the infectivity of VeroE6 cells by SARS-CoV-2 virus.

Another test for the virucidal activity of the composition consists of:
VS10 (virucidal solution 10) consisting of:
- Acetic acid at a concentration of 25 mg/ml (or 2.5% or 2.5 g/100 ml);
- Citric acid at a concentration of 2 mg/ml (or 0.2% or 0.2 g/100 ml);
- Phosphoric acid at a concentration of 0.6 mg/ml (or 0.06% or 0.06 g/100 ml);
- Water.

This test was performed directly on SARS-CoV-2 GT4CNS virus exposed to the composition for 5 minutes and incubated with VeroE6 cells, giving a 75 to 80% reduction in the infectivity of VeroE6 cells by SARS-CoV-2 virus.

These results are summarized in the table below:

| Virucidal Compositio n (VS) | Acetic acid | Sodium acetate | Citric acid | Phosphor ic acid | Sum of acids | Virus | Exposure time (mins) | % of virucidal activity |
|---|---|---|---|---|---|---|---|---|
| VS1 | 52.50% | | 2.00% | 0.6% | 55.10% | MVVA | 1 | 99.99 |
| VS2 | 26.25% | | 1.00% | 0.3% | 27.55% | MVVA | 1 | 9999 |
| VS3 | 10.00% | | 0.4% | 0.12% | 10.52% | MVVA | 1 | 99.99 |
| **VS4** | 5.25% | | 0.2% | 0.06% | **5.51%** | MVVA | 1 | 99.99 |
| VS5 | 0.02% | | 0.001% | 0.0003% | 0.02% | MVVA | 1 | 0 |
| VS6 | 25.00% | | 1.00% | 3.00% | 29.00% | SARS-CoV2 | 15 | 99.99 |
| **VS7** | 5.25% | | 0.2% | 0.06% | **5.51%** | SARS-CoV2 | 5 | 97-99 |
| **VS8** | 2.5% | | 1.75% | 0.06% | **4.31%** | SARS-CoV2 | 5 | 97-99 |
| VS9 | | 7.17% | 0.2% | 0.06% | 0.26% | SARS-CoV2 | 5 | 40 |
| VS10 | 2.5% | | 0.2% | 0.06% | 2.76% | SARS-CoV2 | 5 | 75-80 |

While most of the chewing gum compositions based on the combination of the three acids gave good results in relation to their virucidal capacity, VS8 chewing gum proved to be the one with the best organoleptic results, largely because it contained the least acid.

### Study of the release profile of the chewing gum according to the invention

The chewing gum with VS8 composition consists of:
- Acetic acid at a concentration of 25 mg/ml (or 2.5% or 2.5 g/100 ml);
- Citric acid at a concentration of 17.5 mg/ml (or 1.75% or 1.75 g/100 ml);
- Phosphoric acid at a concentration of 0.6 mg/ml (or 0.06% or 0.06 g/100 ml).

Furthermore, it represents a virucidal activity of 97 to 99% at chewing gum-compatible acid concentrations in a salivation environment of 4 ml/min, providing an experimentally determined release from the base gum of:
- 25% acetic acid per minute;
- 35% citric acid per minute;
- 50% phosphoric acid per minute.

According to the foregoing, the concentrations of the chewing gum components were adjusted to the release values for each of the 3 acids in order to reach the concentrations expressed in VS8 in the oral cavity during the first minute of chewing; therefore, the concentrations of the acids added to the chewing gum were:
- Acetic acid at a concentration of 100 mg/ml (or 10% or 10 g/100 ml);
- Citric acid at a concentration of 50 mg/ml (or 5% or 5 g/100 ml);
- Phosphoric acid at a concentration of 1.2 mg/ml (or 0.12% or 0.12 g/100 ml).

### Toxicity study on human cells

The cytotoxicity of the components on human cells was analyzed in:
THP-1: Human monocytic leukemia cell line obtained from the American Type Culture Collection (ATCC, TIB-202) and cryopreserved in liquid nitrogen. Cells were grown in suspension and maintained in RPMI-1640 medium supplemented with 0.05 mM 2-mercaptoethanol and 10% v/v inactivated fetal bovine serum (Invitrogen).

HL60: Human promyelocytic cell line cultured in RPMI medium in suspension supplemented with 10% inactivated FBS.

A459: Epithelial cell line derived from human basal alveolar adenocarcinoma in RPMI with 10% inactivated FBS.

HepG2: Adherent hepatocellular liver cell line cultured in RPMI supplemented with 10% inactivated FBS.

No differences were observed in the spontaneous release of LDH (cells incubated with medium) and cells incubated with the composition at any of the concentrations analyzed after exposure for up to 120 minutes, the composition did not induce any toxicity in any of the four cell lines. Where the composition consisted of the following solutions:
10X concentrated
   - Acetic acid at a concentration of 600 mg/ml (or 60% or 60 g/100 ml);
   - Citric acid at a concentration of 2 mg/ml (or 0.2% or 0.2 g/100 ml);
   - Phosphoric acid at a concentration of 0.6 mg/ml (or 0.06% or 0.06 g/100 ml);
   - RPMI culture medium (Lonza, BE120-702F).
5X concentrated
   - Acetic acid at a concentration of 300 mg/ml (or 30% or 30 g/100 ml);
   - Citric acid at a concentration of 1 mg/ml (or 0.1% or 0.1 g/100 ml);
   - Phosphoric acid at a concentration of 0.3 mg/ml (or 0.03% or 0.03 g/100 ml);
   - RPMI culture medium (Lonza, BE120-702F).
2X concentrated
   - Acetic acid at a concentration of 120 mg/ml (or 12% or 12 g/100 ml);
   - Citric acid at a concentration of 0.4 mg/ml (or 0.04% or 0.04 g/100 ml);
   - Phosphoric acid at a concentration of 0.12 mg/ml (or 0.12% or 0.12 g/100 ml);
   - RPMI culture medium (Lonza, BE120-702F).
1X concentrated
   - Acetic acid at a concentration of 60 mg/ml (or 6% or 6 g/100 ml);
   - Citric acid at a concentration of 0.2 mg/ml (or 0.02% or 0.02 g/100 ml);
   - Phosphoric acid at a concentration of 0.06 mg/ml (or 0,006% or 0,006 g/100 ml);
   - RPMI culture medium (Lonza, BE120-702F).

Acidic solution made up of three of the following acids: acetic acid, citric acid and phosphoric acid. The compound to be analyzed was prepared concentrated tenfold (10X) (2 mg/mL citric acid, 0.6 mg/mL phosphoric acid and 600 mg/mL acetic acid). The effect of the compound on cell viability was analyzed at four concentrations: 10x, 5x, 2x and 1X. No cytopathic effect was observed on the analyzed cells.

### Study related to the risk of tooth erosion

For the selection of VS8 as an integral chewing gum composition, three parameters related to the risk of tooth erosion and the transience of the decrease in the pH of saliva were additionally measured.

The transience of the decrease in the pH of saliva contained in the oral cavity when chewing the gum was preliminarily established on 6 volunteers by chewing the chewing gum with the virucidal composition (3 volunteers) and with an acid-free commercial chewing gum control (3 volunteers), measuring the pH of the saliva in each volunteer after 0, 2, 4, 8 and 20 minutes of chewing. As shown in the table below, the VS8 solution contained in the chewing gum provides an optimal transience of the decrease in pH, with minimum values of about pH 4 between 2 and 4 minutes, and the restoration of complete neutrality at 20 minutes. This situation is compatible with the virucidal purposes proposed for the composition and dental safety and integrity.

### pH values in saliva

| **Volunteer** | **A** | **B** | **C** | **D** | **E** | **F** |
|---|---|---|---|---|---|---|
| Chewing gum type "VS8" or "C" (control) | VS8 | C | C | VS8 | C | VS8 |
| Chewing time | | | | | | |
| 0 | 7 | 7 | 7 | 7 | 7 | 7 |
| 2 | 5 | 8 | 9 | 4 | 8 | 4 |
| 4 | 7 | 7 | 8 | 6 | 8 | 6 |
| 8 | 6 | 7 | 7 | 6 | 7 | 5 |
| 20 | 7 | 7 | 7 | 7 | 7 | 7 |

For the selection of VS8 as an integral chewing gum composition, three parameters related to the risk of tooth erosion were additionally measured, and two commonly consumed substances (Pepsicola, as an example of a carbonated beverage, and commercial lemon juice) were used as comparators. The parameters were studied at exposure times of 5, 10 and 20 minutes. The enamel layer was mechanically leveled to a homogeneous thickness of 0.04 microns on the bovine tooth portions for the test. The enamel surface changes caused by the VS8 solution and the solutions used as comparators, commercial lemon juice (Jif Lemon Juice Code: L120302M8) and Pepsicola (Pepsi Original Jan 2022 Y06L, 1204), were quantified and the results recorded as set out below for each of the parameters. (Testing conducted entirely at Intertek CRS, Unit A4, Elm House, Oaklands Office Park, Hooton, Cheshire CH66 7NZ, Great Britain.

In the case of the VS8 composition contained in the chewing gum, a 50:50 dilution with artificial saliva was established at 5 and 10 minutes exposure to make the conditions resemble the dilution that occurs in the mouth by chewing the chewing gum with an increased saliva flow of 4 ml/min on average.

The 3 parameters studied were:
1. Surface micro-hardness: this parameter refers to the loss of enamel surface hardness as a result of exposure to a compound. Changes in this parameter are reversible and the affected enamel surface can be recovered with normal saliva flow.
Changes in surface micro-hardness (HK) (*Mean Post-Exposure Changes in SMH Surface Micro-hardness*)

| **Treatment** | **After 5 min (+ SD)** | **After 10 min (+ SD)** | **After 20 min (+ SD)** |
|---|---|---|---|
| Pepsi | -70.45 (+31.71) | -121.93 (+28.27) | -150.53 (+18.33) |
| VS8 | -153.23 (+22.22) | -183.18 (+29.15) | -201.48 (+22.11) |
| Jif Lemon J. | -197.35 (+21.67) | -235.15 (+22.06) | -257.15 (+17.66) |

2. Enamel roughness level: this parameter refers to the changes caused by the compound on the surface structure of the tooth, and measures the smoothness of the tooth surface as a measure for predicting potential damage from exposure to a compound.
Changes in enamel roughness level (Sa) (*Mean Post-Exposure Change in Enamel Surface Roughness*)

| **Treatment** | **After 5 min (+ SD)** | **After 10 min (+ SD)** | **After 20 min (+ SD)** |
|---|---|---|---|
| Pepsi | 0.02443 (±0.02594) | 0.0426 (±0.0474) | 0.3008 (±0.1653) |
| VS8 | 0.1322 (±0.0419) | 0.2083 (±0.0706) | 0.2966 (±0.2184) |
| Jif Lemon J | 0.1882 (±0.0359) | 0.3572 (±0.1987) | 0.5877 (±0.2681) |

3. Enamel erosion: this parameter refers to the actual loss of enamel and is carried out on teeth from which the most superficial protective layer of dentin has been mechanically removed. The objective is to measure the corrosive capacity of a compound directly on the enamel. A positive result in this test does not imply corrosion on a healthy tooth since it has a protective layer and is only an indicator of the compound's potential to attack exposed, and therefore accessible, enamel.

### Enamel erosion (µm) (Mean Post-Exposure Enamel Erosion)

| **Treatment** | **After 5 min (+ SD)** | **After 10 min (+ SD)** | **After 20 min (+ SD)** |
|---|---|---|---|
| Pepsi | 0.0410 (±0.0764) | 0.0370 (±0.0521) | 0,633 (±0,399) |
| VS8 | 0.4887 (±0.2102) | 0,702 (±0,350) | 0,858 (±0,490) |
| Jif Lemon J | 1,106 (±0,378) | 3,156 (±1,234) | 4,669 (±2,451) |

The results obtained from measuring these parameters according to dilution guidelines corresponding to chewing the chewing gum in the mouth, and used for VS8 at exposure times up to 20 minutes indicate that: softening of tooth surface micro-hardness, changes in enamel roughness and enamel erosion are within the safety margins, with statistical validation approaching the effects caused by a commonly consumed carbonated beverage (Pepsicola) and significantly below the effects caused by commercial lemon juice.

## Claims

1. Chewing gum containing:
1 to 30% w/v acetic acid,
0.2 to 10% w/v citric acid,
0.01 to 1.0% w/v phosphoric acid, and
wherein the % w/v is with respect to the weight of the total volume of the composition.

2. Chewing gum according to claim 1, wherein:
acetic acid is included in an amount of 2 to 3% w/v, and is preferably 2.5%;
citric acid is included in an amount of 1.5 to 2% w/v, and is preferably 1.75%; and
phosphoric acid is included in an amount of 0.05 to 0.07% w/v, and is preferably 0.06%.

3. Chewing gum according to claim 2, wherein the chewing gum base comprises:
- elastomeric polymers, preferably specially purified elastomeric polymers;
- resins;
- refined waxes;
- plant fatty acid glycerol esters;
- fully hydrogenated vegetable oils;
- talc;
- antioxidant, preferably TCPHN in a maximum amount of 0.1%.

4. Chewing gum according to any of the preceding claims, comprising a base gum and at least one or more components selected from the group of sweeteners, flavorings, taste-masking agents and vitamins.

5. A chewing gum and wrapper set, wherein the chewing gum is according to any of the preceding claims and the wrapper comprises waxed paper in direct contact with the chewing gum.
